(19) **Europäisches Patentamt**
**European Patent Office**
**Office européen des brevets**

(11) **EP 1 874 366 B1**

(12) **EUROPEAN PATENT SPECIFICATION**

(45) Date of publication and mention of the grant of the patent:
**18.03.2009 Bulletin 2009/12**

(51) Int Cl.:
*A61L 27/40* (2006.01)   *A61L 27/44* (2006.01)
*A61L 27/48* (2006.01)

(21) Application number: **06725946.5**

(22) Date of filing: **27.04.2006**

(86) International application number:
**PCT/FI2006/050171**

(87) International publication number:
**WO 2006/114483 (02.11.2006 Gazette 2006/44)**

(54) **A BIOABSORBABLE AND BIOACTIVE COMPOSITE MATERIAL AND A METHOD FOR MANUFACTURING THE COMPOSITE**

BIOABSORBIERBARES UND BIOAKTIVES VERBUNDMATERIAL UND VERFAHREN ZUR HERSTELLUNG DES VERBUNDMATERIALS

MATERIAU COMPOSITE BIO-ACTIF ET BIO-ABSORBABLE ET PROCEDE DE FABRICATION DE CE COMPOSITE

(84) Designated Contracting States:
**AT BE BG CH CY CZ DE DK EE ES FI FR GB GR HU IE IS IT LI LT LU LV MC NL PL PT RO SE SI SK TR**

(30) Priority: **27.04.2005 FI 20055194**

(43) Date of publication of application:
**09.01.2008 Bulletin 2008/02**

(73) Proprietor: **BIORETEC OY**
**33720 Tampere (FI)**

(72) Inventors:
• **TÖRMÄLÄ, Pertti**
**FI-33300 Tampere (FI)**
• **HUTTUNEN, Mikko**
**FI-33180 Tampere (FI)**
• **ASHAMMAKHI, Nureddin**
**Staffordshire ST4 6BY (GB)**
• **TUKIAINEN, Mikko**
**FI-33720 Tampere (FI)**
• **YLÄNEN, Heimo**
**FI-20810 Turku (FI)**

• **HUPA, Mikko**
**FI-20720 Turku (FI)**
• **KELLOMÄKI, Minna**
**36200 Kangasala (FI)**

(74) Representative: **Hakola, Unto Tapani**
**Tampereen Patenttitoimisto Oy**
**Hermiankatu 1 B**
**33720 Tampere (FI)**

(56) References cited:
**WO-A-96/00592          WO-A-99/11296**

• **HUANG Z-M ET AL: "Stiffness and strength design of composite bone plates" COMPOSITES SCIENCE AND TECHNOLOGY, ELSEVIER, vol. 65, no. 1, January 2005 (2005-01), pages 73-85, XP004619375 ISSN: 0266-3538**
• **TÖRMÄLÄ P: "Biodegradable self-reinforced composite materials; manufacturing structure and mechanical properties." CLINICAL MATERIALS. 1992, vol. 10, no. 1-2, 1992, pages 29-34, XP002400093 ISSN: 0267-6605 cited in the application**

**Description**

Field of the invention

The present invention relates to a bioabsorbable and bioactive composite material for surgical musculoskeletal applications comprising a polymeric matrix material which is reinforced with bioabsorbable polymeric fibers and bioabsorbable ceramic fibers.

Background of the Invention

Biostable or bioabsorbable devices are used in surgery for musculoskeletal applications, such as e.g. (a) screws, plates, pins, tacks or nails for the fixation of bone fractures and/or osteotomies to immobilize the bone fragments for healing, (b) suture anchors, tacks, screws, bolts, nails, clamps and other devices for soft tissue- to- bone (or- into- bone) and soft tissue-to-soft tissue fixation or (c) cervical wedges and lumbar cages and plates and screws for vertebral fusion and other operations in spinal surgery.

Most biostable devices are typically made of metallic alloys (see e.g. M.E. Müller, M. Allgöwer, R. Schneider, H. Willenegger "Manual of Internal Fixation", Springer-Verlag, Berlin Heidelberg New York 1979). However, there are several disadvantages in the use of metallic implants. One such disadvantage is bone resorption caused by high modulus bone plates and screws, which carry most of the external loads, leading to stress protection produced by the modulus mismatch between metals and bone. Another disadvantage is the possibility of corrosion. Therefore, it is recommended that surgeons should remove metallic devices (like bone plates and screws) in a second operation once the fracture has healed.

Bioabsorbable polymeric fracture fixation devices have been developed and studied as replacements for metallic implants (see e.g. S. Vainionpää, P. Rokkanen, P. Törmälä, "Surgical Applications of Biodegradable Polymers in Human Tissue", Progress in Polymer Science, Vol. 14, 1989, pp. 679-716). The advantages of these devices are that materials are resorbed in the body and the degradation products exit via metabolic routes. Hence, a second operation is not required. Additionally, the strength and the stiffness (modulus) of the bioabsorbable polymeric devices decreases when the device degrades and hence the bone is progressively loaded more and more, which promotes bone regeneration (according to Wolff's law).

One limitation of the prior art bioabsorbable materials and devices is their relatively low modulus and strength. In the case of cortical bone fractures, for example, non-reinforced poly lactic acid (PLLA) plates and screws are initially too weak to permit patient mobilization ( see e.g. J. Eitenmüller, K.L. Gerlach, T. Schmickal, H. Krause, "An in Vivo Evaluation of a New High Molecular Weight Polylactide Osteosynthesis Device", European Congress on Biomaterials, Bologna Italy, September 14-17, 1986, p. 94).

Törmälä et al. have developed self-reinforced bioresorbable polymeric composites to improve the strength of bioresorbable polymer devices. These show relatively good mechanical properties: e.g. bending strength of $360\pm70$ MPa and bending modulus of $12\pm2$ GPa, respectively, have been reported (see P. Törmälä, "Biodegradable Self-Reinforced Composite Materials; Manufacturing, Structure and Mechanical Properties", Clinical Materials, Vol. 10, 1992, pp. 29-34). However, the reported modulus values are still below the modulus values of strong cortical bone (see e.g. S.M. Snyder and E. Schneider, "Estimation of Mechanical Properties of Cortical Bone by Computed Tomography", Journal of Orthopedic Research, Vol. 9, 1991, pp. 422-431, giving the bending modulus of 17.5 GPa for human tibial bone). It is desirable that the modulus of a fixation device is at least as high as the modulus of cortical bone so that the fixation system is practically isoelastic with the bone, which gives the possibility to natural, controlled micromotions of fixed bone fragments in relation to each other. Such natural micromotions accelerate the fracture consolidation and ossification (healing) and reduce the risks of too big micromotions (leading to fibrous non-union) or too small micromotions (leading to stress-protection atrophy and increased porosity of healing bone).

A common property of most polymeric implants is the lack of bony ongrowth to the materials. In contrast, such bone apposition is produced by bioactive ceramics, such as bioactive glasses (see e.g. O.H. Andersson, K.H. Karlsson, "Bioactive Glass, Biomaterials Today and Tomorrow", Proceedings of the Finnish Dental Society Days of Research, Tampere, Finland, 10-11 November 1995, Gillot Oy, Turku, 1996, pp. 15-16). By adding (compounding) bioactive ceramics, such as bioactive glasses to polymers to produce composites, the bioactivity of the material can be improved. This effect has been demonstrated in dental composites and bone cement (see e.g. J.C. Behiri, M. Braden, S.N. Khorashani, D. Wiwattanadate, W. Bonfield, "Advanced Bone Cement for Long Term Orthopaedic Applications", Bioceramics; Vol. 4, ed. W. Bonfield, G.W. Hastings and K.E. Tanner, Butterworth-Heinemann Ltd., Oxford, 1991, pp. 301-307).

Zimmerman et al. developed unidirectional composites of poly-L-lactide matrix reinforced with calcium / phosphorous oxide (CaP) based biodegradable glass fibers. This composite showed good initial strength and modulus values, e.g. tensile strength $200.3\pm7.1$ MPa, tensile modulus $29.9\pm2.2$ GPa, bending strength $161.3\pm8.8$ MPa, bending modulus $27.0\pm0.3$ GPa. However, the strength reinforcing effect of CaP fibers in hydrolytic conditions (in vitro: a tris-buffered saline of pH 7.4 at 37°C) was lost totally after 23 days of immersion, while only 35 % of the initial strength and 45 % of the initial modulus was retained. It can be concluded that this composite, which was reinforced with long ceramic fibers, lost its strength too rapidly to be applied as a raw material of bone fracture fixation devices. See M. Zimmerman, T. Guastavin, J.R. Parsons, H. Alexander and T.C. Lin: "The in vivo biocompatibility and in vitro degradation of absorbable glass fiber reinforced composites", 12th Ann. Meeting Soc. Biomater., p. 16, Minneapolis-St.Paul, Minnesota, USA (1986).

A. Saikku-Bäckström et al. Studied *in vivo* and *in vitro* hydrolysis of poly-96L/4D-lactide fiber reinforced poly-96L/4D-lactide matrix (fibrillated SR-PLA 96) rods (diam. 1.1 mm). The rods had an initial bending strength of 225 MPa and a bending modulus of 8.4 GPa. After 168 days (24 wk) of *in vitro* hydrolysis in buffered saline at 37°C, the bending strength was still 86.7 % (195 MPa) of the initial value. The bending modulus of same rods after 168 days hydrolysis in the above conditions was still 82.1 % (6.9 GPa) of the initial value. It can be concluded that these bioabsorbable polylactide fiber reinforced polylactide rods had a good bending strength and a bending modulus retention in hydrolytic conditions, even if the initial bending modulus was still far below the bending modulus of cortical bone. See: A. Saikku-Bäckström et al. in J. Mater. Sci: Mater. Med. 10 (1999) p. 1-8.

Bioabsorbable composites of hydroxyapatite and copolymers of polyhydroxybutyrate and polyhydroxyvalerate have been described by C. Doyle, K.E. Tanner, W. Bonfield, see "In Vitro and in Vivo Evaluation of Polyhydroxybutyrate and of Polyhydroxyvalerate Reinforced with Hydroxyapatite", Biomaterials, Vol. 12, 1991, pp. 841-847). The main limitation of these bioabsorbable composites is their inadequate mechanical strength for large bone fracture fixation. Also, the use of hydroxyapatite and polylactic acid composites has been reported. See Y. Ikada, H.H. Suong, Y. Shimizu, S. Watanabe, T. Nakamura, M. Suzuki, A.T. Shimamoto, "Osteosynthetic Pin", U.S. Patent 4,898,186, 1990. Using existing elements the composites still have quite moderate mechanical strength and modulus.

Prior art also teaches biodegradable and bioactive composites with at least one resorbable polymeric reinforcing element and at least one ceramic reinforcing element with a particle size between 2μm and 150μm (see P. Törmälä, M. Kellomäki, W. Bonfield, K.E. Tanner, "Bioactive and Biodegradable Composites of Polymers and Ceramics or Glasses and Method to Manufacture such Composites", EP 1 009 448 B1). Even if these composites show improved strength and modulus in comparison to many non-reinforced bioactive polymer - ceramic composites, their modulus values (8.3 GPa - 14.2 GPA) are still clearly lower than the modulus values of strong cortical bone (see e.g. Snyder and Schneider above).

Q.-Q. Qin et al. describe in WO 2004049904 a flexible, bioactive mesh comprising glass fibers and first resorbable polymer fibers in which said glass fibers are interwoven with said first resorbable polymer fibers. However, this is a low modulus material because there is no matrix polymer which could transfer loads from fibers to each other and could prevent fibers from moving in relation to each other when external forces are directed to the mesh.

Accordingly, prior art teaches that (a) bioabsorbable composites, reinforced with absorbable glass fibers, have a high initial bending modulus, but they rapidly lose their strength and modulus *in vitro,* and (b) bioabsorbable composites reinforced with bioabsorbable polymer fibers have a good strength retention *in vitro,* but their initial bending modulus values are well below the modulus values of cortical bone, and (c) bioabsorbable composites reinforced with bioabsorbable polymer fibers and with ceramic reinforcing elements with a particle size between 2 μm and 150 μm, also have initial bending modulus values below the modulus values of cortical bone.

Accordingly, there exists a need for strong bioabsorbable, composite materials with high initial bending modulus and bending strength to guarantee the safe initial consolidation and healing of bone fractures. There exists further a need for such materials which additionally retain the high strength values under hydrolytic conditions at 37°C over four weeks to guarantee the safe consolidation and healing of bone fractures. There exist further a need for such materials, which additionally are osteoconductive, which means that they promote and facilitate bone healing.

Such materials with high initial modulus and good strength retention *in vitro* are useful in manufacturing of e.g. bone fracture fixation devices, because high initial modulus and strength retention under hydrolytic conditions provide the devices with an initial isoelastic behaviour in comparison to the healing bone, which means stronger control of micromotions in the healing bone, leading to an improved healing and to a lower risk of non-unions during healing. The high strength of the implant guarantees safe progress of healing after the early consolidation of the fracture.

**Summary of the invention**

Now, we have surprisingly found that bioabsorbable, bioactive composites with the high initial modulus and strength (specially high impact strength) and good strength retention behaviour *in vitro* under hydrolytic conditions are obtained by reinforcing a bioabsorbable polymer matrix both with bioabsorbable polymeric fibers and with bioabsorbable ceramic fibers, of which at least a portion is longer than 150 μm.

We shall describe composite materials of the invention, which comprise at least one polymeric matrix phase, at least one bioactive ceramic reinforcing long fiber phase and at least one bioabsorbable polymeric reinforcing long fiber phase as defined in claim 1. The reinforced composite materials described in this invention have an improved combination of mechanical strength and modulus properties when compared to reinforced and non-reinforced materials and devices of prior art, because reinforcement with long ceramic and polymeric fibers will increase both the modulus and strength retention of the material when compared to prior art materials. Thanks to the controlled manufacturing stages of combining of matrix and ceramic reinforcing fibers as well as polymeric reinforcing fibers, the amount of both reinforcing fiber types can be easily controlled. This is an advantage, because the ratio of the elements will affect the mechanical properties of the device. Also, the amount of the ceramic reinforcing fibers will affect the bioactivity of the device.

Bioabsorbable polymeric long fibers and ceramic fibers differ significantly from each other in their mechanical behaviour.

Polymeric long fibers are tough and strong, and therefore they can increase the toughness and strength values, such as the tensile, bending and impact strength of composites.

Ceramic long fibers have high stiffness and therefore they can increase the stiffness (modulus values) of even polymer fiber reinforced composites.

By combining long bioabsorbable polymeric fibers and long ceramic fibers in different ways with the bioabsorbable polymer matrix, we can obtain bioabsorbable high modulus composites with different property combinations.

Reinforcing of the bioabsorbable polymer matrix both with bioabsorbable, polymeric long fibers and with bioabsorbable ceramic long fibers will provide the materials with unique properties: for example, when a fixation implant (e.g. a pin or a screw) for a bone fracture is made of this material, the implant has first a high strength and modulus when both polymeric and ceramic fibers reinforce the implant. This means that the fixation implant is secure and gives an optimal protection for the early consolidation of the bone fracture. Thereafter, typically after some weeks, the ceramic fibers will lose their reinforcing effect, so that only bioabsorbable fibers reinforce the matrix. As a consequence, the strength and the modulus of the implants decrease progressively. However, this decreasing is not as drastic as in prior art materials, since bioabsorbable reinforcing fibers still maintain the strength and ductility of the implant, typically up to 2-6 months after the implantation. This secures the final healing of bone fractures for which the ceramic fibers gave the early strong protection for early consolidation.

Besides the long polymeric fibers and long ceramic fibers, the composite of the invention may include bioabsorbable ceramic fibers having a length which is 150µm or shorter, or bioabsorbable ceramic powder.

In addition to the above-mentioned findings, we shall describe laminates which are reinforced with the ceramic fibers and the polymeric fibers.

**Brief Description of the Drawings**

Figures 1a, 1b and 5 show schematic perspective views of typical composite materials of the invention,

Figures 2 and 3 show schematic cross-sectional views of composite materials of the invention,

Figure 4 shows a perspective view of the cage-like embodiment of the invention,

Figure 6 shows schematically a formation of a laminate material according to an embodiment of the present invention,

Figure 7 shows the 3-point bending strength and the bending modulus as a function of the amount of the bioabsorbable glass fibers,

Figures 8 - 10 show structures of laminate materials in a perspective view, and

Figure 11 shows results of IZOD impact strength measurements.

**Description of the Preferred Embodiments**

The present invention relates to bioabsorbable and bioactive composite materials as defined in clam 1. Said materials are useful for musculoskeletal applications, such as e.g. bone fracture or osteotomy fixation, soft tissue (like tendon)- to -bone fixation, soft tissue - to - soft tissue fixation and guided bone regeneration applications, such as vertebral fusion. Unlike other materials used in prior art, the composites of this invention have two different reinforcing, bioabsorbable, long fiber phases and at least one matrix phase. One reinforcing long fiber phase is referred to as the polymeric reinforcing fiber phase and the other as the ceramic reinforcing fiber phase. The matrix component can be any bioabsorbable or bioerodible polymer, copolymer or polymer alloy (mixture of two or more polymers or copolymers). The polymer can be synthetic or "semisynthetic", which means polymers made by chemical modification of natural polymers (such as starch). Typical examples of polymers, which can be used in this invention, are listed in Table 1 below.

The polymeric reinforcing fibers and ceramic reinforcing fibers are recognizable and distinguishable in the final product.

The diameter of the reinforcing polymeric long fibers can vary typically between 4 $\mu$m and 800 $\mu$m, preferably between 20$\mu$m and 500 $\mu$m. The most useful range is between 30 $\mu$m and 70 $\mu$m. Useful polymers for the polymeric reinforcing fibers include several of those listed in Table 1.

The ceramic reinforcing fibers typically comprise biodegradable, bioactive long fibers of bioactive glass with diameters typically from 1 $\mu$m to 800 $\mu$m and preferably from 5 $\mu$m to 500 $\mu$m. The diameters of ceramic reinforcing fibers are often in the range between 1 $\mu$m and 20 $\mu$m. The fibers with a diameter less than 10 $\mu$m, are of importance. Typical examples are listed in Table 2. They can be used as long single fibers, as yarns, braids, bands or as different types of fabrics made by the methods of textile technology.

Ceramic fibers and/or polymeric fibers may also be introduced in the polymer or composite structure in the form of prefabricated products, such as prepregs, etc., manufactured by means of techniques of the polymer composite technology, in addition to the methods of textile technology.

The fibers of this invention are long, which means that their length is many times (10 x or more) their diameter. They are typically longer than 150 $\mu$m, preferably longer than 2 millimeters and more preferably longer than 30 millimeters. At their best, the fibers are continuous so that their length is the same (or greater) than the longest dimension of the device (the fibers can be longer than the longest dimension of the device if the fibers are e.g. twisted, wound or braided).

The ceramic reinforcing fibers also act as a bioactive, bony ongrowth agent and provide a reservoir of calcium and phosphate ions, thus accelerating the bone healing. These ions may also have a buffering effect on the acidic degradation products of the resorbable polymeric components of the composite. While the matrix polymer degrades, bone can attach to the residual ceramic or glass material. The amount of polymeric reinforcing fibers or ceramic reinforcing fibers in the composite is from 10wt% to 90 wt%, preferably from 20wt% to 70 wt%.

The materials of the invention may contain various additives and modifiers which improve the performance of the composite or its processability. Such additives include surface modifiers to improve the attachment between the polymeric and ceramic components. The devices may also contain pharmaceutically active agents, such as antibiotics, chemotherapeutic agents, wound-healing agents, growth hormones and anticoagulants (such as heparin). These agents are used to enhance the bioactive feature of the composite, to make it multifunctional and to improve the healing process of the operated tissues.

Manufacturing of the composite can be performed by any suitable processing methods of plastics technology, polymer composite technology and/or textile technology. The matrix polymer and the polymeric reinforcing fibers and the ceramic reinforcing fibers (bioceramic or bioactive glass) can be mixed together by mechanical mixing, melt mixing or solvent mixing. The polymeric and/or ceramic reinforcing fibers can be used as plain fibers or in a modified form: for example, as braided, knitted or woven to two - or three - dimensional structures (together or as separate fabrics) or in the form of preforms such as prepregs. The mixture of matrix and the polymeric reinforcing fibers and the ceramic reinforcing fibers can be combined by mixing, by coating or by using a solvent as an intermediate to preform the material (prepreg). The material preform or final device can be produced by various techniques including compression molding, transfer molding, filament winding, pultrusion, melt extrusion, mechanical machining or injection molding to any desired shape.

When the polymeric and/or ceramic long reinforcement fibers of the composites of the invention are continuous the composites have better mechanical properties than short or non-continuous long fibre reinforced bioabsorbable composites. One of the most important factors is thus the absence of fiber ends in the continuous fiber reinforced composites, which are sites for crack iniatiation during fracture due to mechanical loading. Processing methods include e.g.:

- postpregging methods
- prepregging methods:

    ■ film stacking
    ■ incapsulated powder impregnation
    ■ melt impregnation
    ■ powder impregnation
    ■ co-weaving
    ■ comingling

The post- and prepregs are placed in controlled orientation during the manufacture of the composite. Next, pressure and heat are applied, resulting in the total or partial melting of the bioabsorbable matrix and the forming of the composite structure after cooling. Continuous bioabsorbable polymer and ceramic fiber reinforced composites can be produced e.g. by compression molding, thermoforming, filament winding, tape laying, braiding and pultrusion methods and by several combinations of these methods. Such methods are disclosed e.g. in the publication (Doctoral Thesis): E. Suokas, "Processing, microstructure and properties of thermotropic liquid crystalline polymers and their carbon fibre composites", Tampere University of Technology, Publication 267, Tampere, Finland 1999, 269 pp.

Due to controlled manufacturing stages of mixing and combining of the matrix and the ceramic reinforcing fibers as well as combining the polymeric reinforcing fibers, the amount of both reinforcing fiber types can be easily controlled. This is an important advantage, because the ratio of the polymeric and ceramic fibers affects the mechanical strength and modulus properties of the device. Also, the amount of the ceramic reinforcing fibers affects the bioactivity of the device. There should be a sufficient amount of bioceramic or bioactive glass fibers to yield bony on - and ingrowth.

Fiber reinforced composite devices described in this invention have improved mechanical properties compared to non-reinforced devices, because reinforcement will change the behavior of the materials from brittle to ductile and thus make the reinforced device more reliable under loading. This feature is very important for load bearing applications, such as bone fracture fixation devices. For example, non-reinforced polylactic acid devices typically have three-point bending strengths of 35-40 MPa and modulus of 3.5-4.0 GPa, and particulate reinforced (hydroxyapatite) polylactic acid devices have values of 25-30 MPa and 5.0 GPa, respectively. When the composite is made of three components: polymer matrix, reinforcing polymer fibers and ceramic particulate filler, the modulus can be increased up to 8-10 GPa (M. Kellomäki et al., 13th Eur. Conf. Biom., Abstracts, Gothenburg, Sweden, Sept. 4-7, 1997, p. 90). However, using long polymer fiber reinforcement and long ceramic fiber reinforcement, under the present invention, the strength and modulus values of composites can still be increased to a significant extent.

One useful bioabsorbable and bioactive composite is a laminate comprising at least two layers. The composite may comprise

- polymeric layers which are not reinforced,
- polymeric layers comprising reinforcing fibers, or
- layers of ceramic fibers.

The polymeric layers, which are not reinforced, may comprise for example poly-L/DL-lactide 70/30. The polymeric layers, which comprise reinforcing fibers, may also comprise for example poly-L/DL-lactide 70/30. One polymeric layer may comprise both ceramic and polymeric fibers, or only ceramic or polymeric fibers. The reinforcing fibers are usually continuous but they can be staple fibers as well. It is also possible that the staple fibers are spun to a yarn which is used for reinforcing in a continuous form.

The fiber orientation in the polymeric layer can vary. The reinforcing fibers can be parallel or traverse to the longitudinal axis of the polymeric layer or they may form an angle with the longitudinal axis. A random orientation is also possible.

The layers of ceramic fibers, such as bioactive glass fibers, may be formed of parallel fibers which are adhesively attached to each other.

Besides the above-mentioned variations, the reinforcing fibers may form textile structures, such as braidings or woven fabrics.

The fiber orientation in the superimposed layers may differ from each other. In such a manner structures, which are strong to all directions, will be produced. Thus the structures resist very well torsional forces.

The layers of the laminate are laminated together by using heat and pressure. The number of the layers to be laminated together varies depending on the desired end use. Those laminates are useful for example in surgical fixation devices, such as fixation plates for bone fractures, or in implants for ossifying vertebrae.

TABLE 1. *Bioabsorbable, (resorbable) polymers, copolymers and terpolymers suitable for composites of the invention (Useful as materials for the bioabsorbable polymeric fibers and for the bioabsorbable polymeric matrix).*

- Polyglycolide (PGA)
- Copolymers of glycolide:

    Glycolide/L-lactide copolymers(PGA/PLLA)
    Glycolide/trimethylene carbonate copolymers (PGA/TMC)

- Polylactides (PLA)
- Stereocopolymers of PLA:

Poly-L-lactide (PLLA)
Poly-DL-lactide (PDLLA)
L-lactide/DL-lactide copolymers

- Other copolymers of PLA:

Lactide/tetramethylglycolide copolymers
Lactide/trimethylene carbonate copolymers
Lactide/d-valerolactone copolymers
Lactide/ε-caprolactone copolymers

- Terpolymers of PLA:

Lactide/glycolide/trimethylene carbonate terpolymers
Lactide/glycolide/ε-caprolactone terpolymers
PLA/polyethylene oxide copolymers

- Polydepsipeptides
- Unsymmetrically 3,6-substituted poly-1,4-dioxane-2,5-diones
- Polyhydroxyalkanoates:

Polyhydroxybutyrates (PHB)
PHB/b-hydroxyvalerate copolymers (PHB/PHV)

- Poly-b-hydroxypropionate (PHPA)
- Poly-p-dioxanone (PDS)
- Poly-d-valerolactone
- Poly-e-caprolactone
- Methylmethacrylate-N-vinyl pyrrolidone copolymers
- Polyesteramides
- Polyesters of oxalic acid
- Polydihydropyrans
- Polyalkyl-2-cyanoacrylates
- Polyurethanes (PU)
- Polyvinylalcohol (PVA)
- Polypeptides
- Poly-b-malic acid (PM LA)
- Poly-b-alkanoic acids
- Polycarbonates
- Polyorthoesters
- Polyphosphates
- Polyanhydrides

TABLE 2. *Bioceramics and glasses suitable for composites of the invention.*

- Hydroxyapatite
- Calcium phosphates:

Tricalcium phosphates

- Bioactive glasses
- Bioactive glass-ceramics

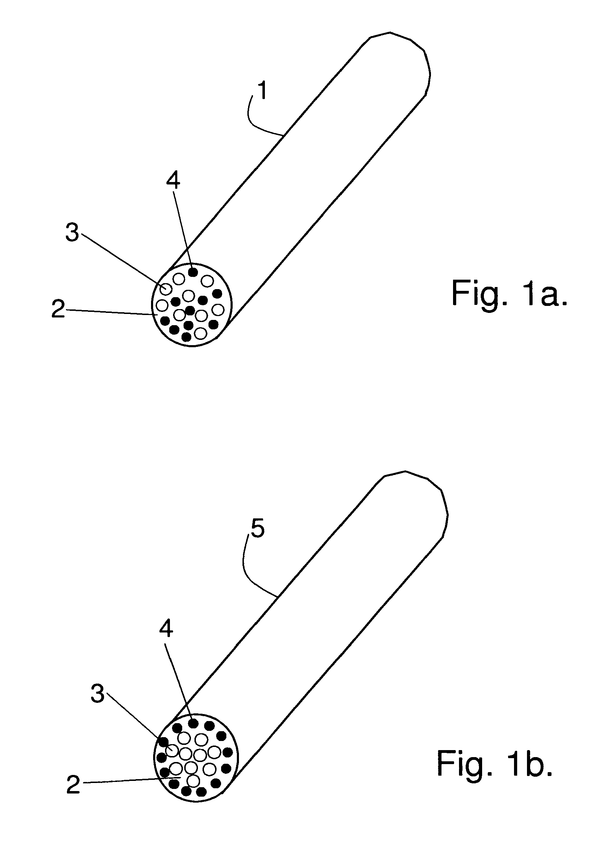

Figure 1a shows a cylindrical bar 1 comprising a polymer matrix 2, long polymer fibers 3 and slightly thinner ceramic fibers 4 bound by the polymer matrix 2.

Figure 1b shows a high bending modulus cylindrical bar 5 with polymer fibers 3 in the core of the bar and ceramic fibers 4 in the surface area of the bar.

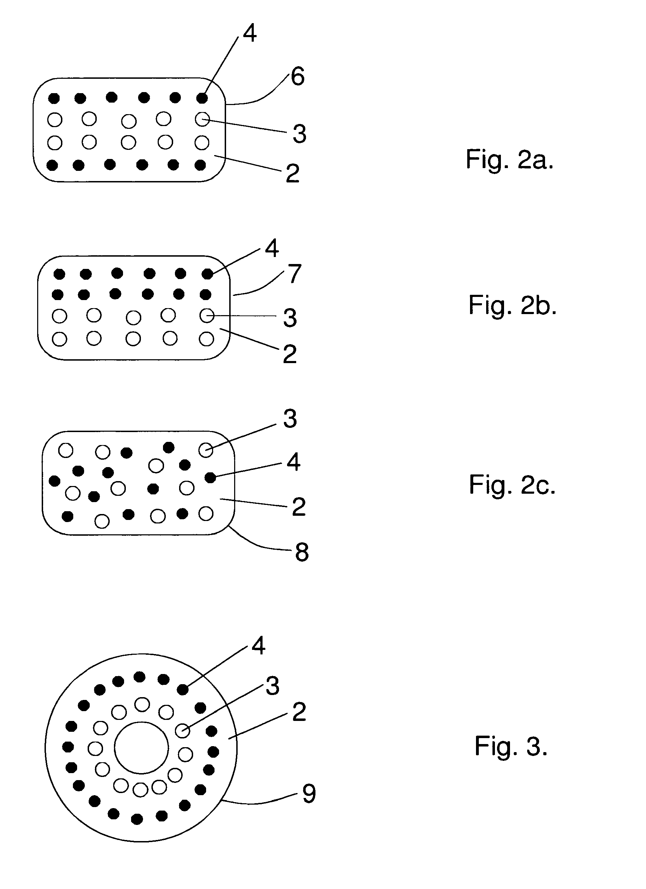

Figure 2 illustrates, as an example, cross-sections of rectangular bars with different arrangements of long bioabsorbable polymeric and ceramic reinforcement fibers. Figure 2a shows the cross-section of a bar 6 with a polymer matrix 2, in which polymeric fibers 3 are found in the inner area of the bar 6 and ceramic fibers 4 near the surfaces of the bar 6.

Figure 2b shows a cross-section of a bar 7 with the matrix 2, in which polymeric fibers 3 are found near the lower surface of the bar 7 and ceramic fibers 4 near the upper surface of the bar 7.

Figure 2c shows a cross-section of a bar 8 with the matrix 2, in which polymer fibers 3 and ceramic fibers 4 are distributed randomly into the matrix 2 of the bar.

Figure 3 illustrates a cross-section of a tubular implant 9 with a parallel, continuous fiber reinforcement by polymeric fibers 3 and ceramic fibers 4, both embedded in polymer matrix 2, according to the invention.

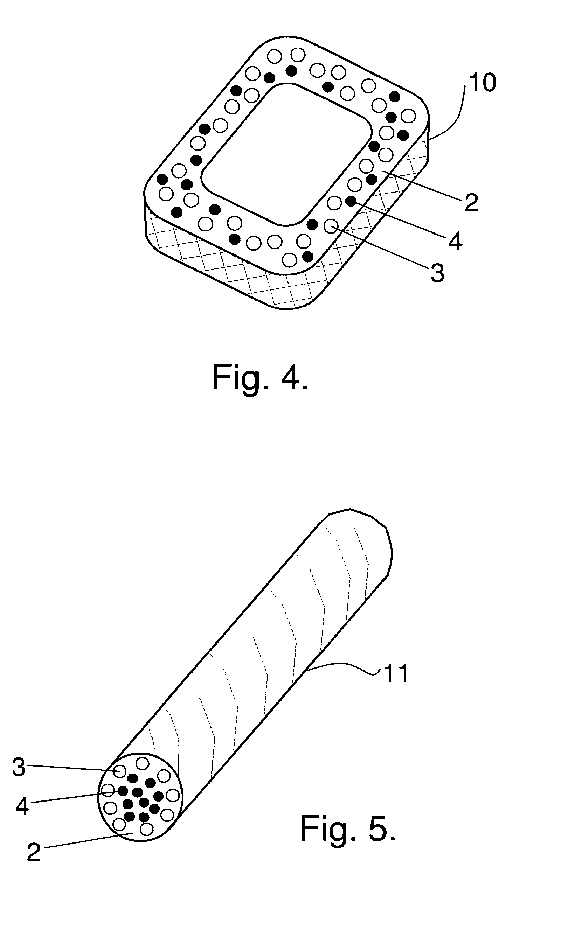

Figure 4 illustrates an advantageous embodiment of the invention. Here a bioabsorbable spinal cage 10 has been reinforced with bioabsorbable polymer fibers 3 and ceramic fibers 4 embedded in a polymer matrix 2. The cage has been made by (a) winding a prepreg of matrix polymer, which is reinforced with continuous polymer and ceramic fibers, around a rectangular mold, (b) by melting the matrix polymer during the winding, and (c) by cooling the composite. Thereafter, the mold is removed from the inside of the rectangular composite tube and the tube has been cut to shorter cage samples.

Figure 5 shows a perspective view of a cylindrical bar 11 of the invention, comprising a polymer matrix 2 and spirally wound polymer fibers 3 and ceramic fibers 4 embedded therein.

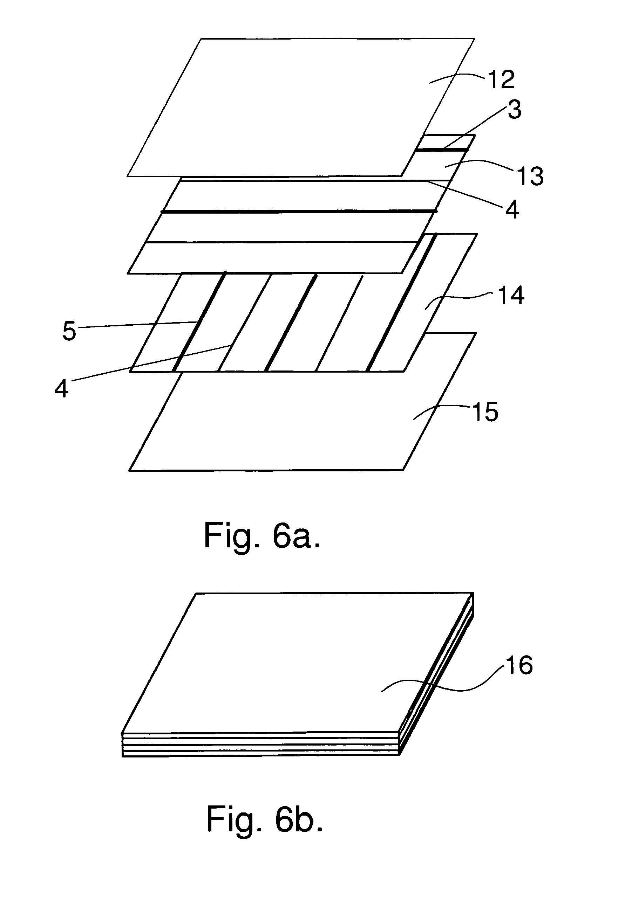

Figure 6 shows a perspective view of a stack of 4 layers: an upper film 12 made of a matrix polymer, a polymeric prepreg 13 including polymer fibers 3 and ceramic fibers 4, a second polymeric prepreg 14 with polymeric fibers 3 and ceramic fibers 4 and a lower film 15. The films and prepregs can be compressed to a composite plate of the invention by using heat and pressure so that the upper and lower films 12 and 15 as well as the matrix of prepregs 13 and 14 melt at least partially and bind the polymeric and ceramic fibers 3 and 4 together to form a polymer matrix plate 16 (of Fig. 6b) reinforced with both polymer fibers and ceramic fibers.

Instead of matrix films it is also possible to use a matrix as fiber fabrics and to melt the fiber matrix to bind polymer and ceramic reinforcing fibers together.

Naturally, the composite materials of the invention can be fabricated by many other methods known in the polymer technology and/or in the composite technology as well as in the textile technology. For example, one advantageous method is injection molding, in which the polymer fiber + a ceramic fiber insert is located inside the mold and a polymer melt is injected into the mold to fill the pores inside the fiber insert and the possible open space around the insert. Thereafter, the mold is cooled down so that the polymer melt (matrix) becomes solid and the composite sample can be removed from the mold.

The composite samples, such as membranes, meshes, foils, plates, rods or tubes, can be applied as implants in tissue fixation, regeneration or tissue generation.

The composite samples can be processed further mechanically and/or thermally into the form of more sophisticated implants to obtain e.g. screws, plates, nails, tacks, suture anchors, bolts, clamps, wedges, cages, etc. to be applied in different disciplines of surgery for tissue management, such as tissue fixation, or to help or guide tissue regeneration and/or generation.

## EXAMPLES

The following non-limiting examples give detailed information about the present invention.

### Example 1.

**Matrix: Poly-L/DL-lactide** 70/30 (PLA$_{70}$), raw material from Boehringer Ingelheim, Germany (RESOMER®LR 708, Lot No. 290358, initial Mw ca. 370 000 Da (I.V.5.9-6.2 dl/g; when processed into form of flat strips MW ca. 215 000 Da) **Polymer fiber-reinforcement:** Poly-L/D-lactide 96/4 raw material from Purac Biochem, the Netherlands (PURASORB®

PLD, Lot No. 0209000939, initial I.V.5.48 dl/g; when processed into form of fibers Mw ca. 150 000 Da). The fibers with final diameter of ca. 85-95 $\mu$m were made by melt spinning with a single screw extruder.

**Glass fiber reinforcement:** Bioactive Glass 1-98 (53.0% $SiO_2SiO_2SiO_2$ 6.0% $Na_2O$, 22.0% CaO, 2.0% $P_2O_5$, 11.0% $K_2O$, 5.0% MgO, 1,0 %, $B_2O_3$),. Bioactive glass fibers with the diameter of ca. 20-35 $\mu$m were manufactured at Tampere University of Technology (Institute of Biomaterials) by glass melt spinning.

**Polymer reinforcement used to bind BaG-fibers: PLGA 50/50,** raw material from Boehringer Ingelheim, Resomer® RG 503, Lot No. 10044449, I.V. 0,41 dl/g.

Test specimens, sized about 50 x 10 x 1.5 mm were manufactured by means of compression molding from preprocessed $PLA_{70}$ flat strips (44-53 wt-%), bioactive glass 1-98 (BaG) fibers (37- 48 wt-%) and $PLA_{96}$ fibers (8-10 wt-%). $PLA_{70}$ flat strips were manufactured by extrusion and they acted as a matrix material. BaG (1-98) fibers were manufactured into a form of prepreg material during glass fiber processing binding them together with PLGA 50/50 (dissolved in acetone). The thickness of the prepregs was about 0.2 to 0.3 mm. The prepreg material was used as a reinforcement including the ceramic reinforcement component having unidirectional fiber alignment. 4-filament $PLA_{96}$ bundles were manufactured by means of fiber spinning, and they were further processed into the form of circular braids. These circular shaped braids were used as a continuous polymer fiber reinforcement covering $PLA_{70}$ flat strips. This means that the longest fibers covered the whole length of the final product. Finally all of these preforms were put into a mold (size 10 mm x 50 mm) in a specific order:

**(BaG**/($PLA_{96}$/$PLA_{70}$/$PLA_{96}$)/**BaG**/($PLA_{96}$/$PLA_{70}$/$PLA_{96}$)/**BaG)**

After that the mold was heated to the desired temperature (139°C to 141°C) using a holding pressure of 5 MPa. When the desired temperature was achieved (typically after 3-5 min), the pressure was raised to the final value of 10 MPa and the mold was kept under heat and pressure for a prespecified time (1 min 30 s). After that the mold was cooled by using water cooling system.

Three point bending strength and modulus was measured for the test specimens using Instron 4411 Materials testing machine (Instron Ltd., High Wycombe, England). Pure $PLA_{70}$ without any reinforcing components was used as a reference material to the manufactured composites. The reference materials were extruded $PLA_{70}$ flat strips having the dimensions of about 50 x8.3x1.5mm.

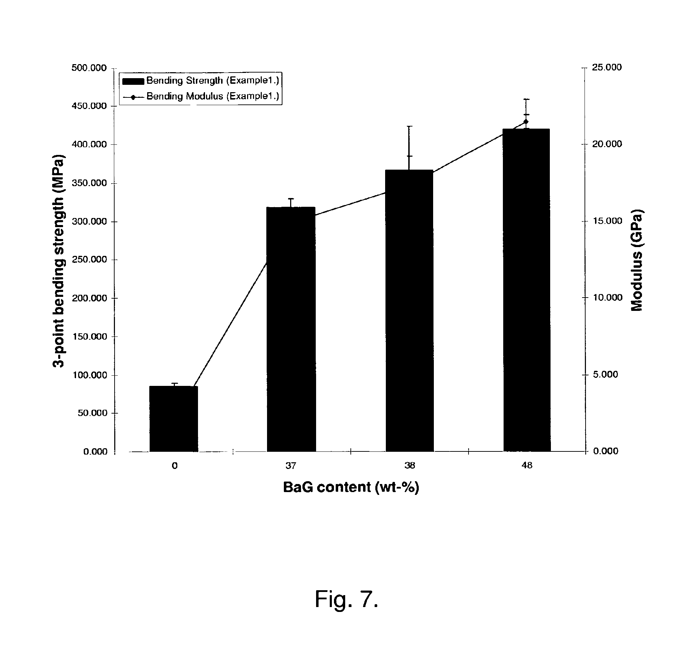

The maximum bending strength (yield) for the manufactured composites was 318.4 to 420.0 MPa and modulus 14.9 to 21.5 Gpa, depending on the BaG-fiber content. In comparison, the bending strength (yield) and modulus for pure $PLA_{70}$ were only 49.4 MPa and 2.2 GPa, respectively. Typical mechanical properties in 3-point bending test for the specimens of Example 1. are shown in Table 1. and in Figure 1. The test specimens expressed fractures shortly after the maximum load, but the tough polymer reinforcement fibers prevented the fragmentation of the samples. Six parallel samples of pure $PLA_{70}$ and two parallel samples of other compositions were studied. Fig. 7 shows 3-point bending properties for manufactured composites. Error bars in Fig. 7 show standard deviations of the measurements.

**Table 1.**

| BaG fiber content wt-% | $PLA_{70}$ matrix content wt-% | $PLA_{96}$ fiber content wt-% | Load at Yield (Max load) (N) | Stress at Yield (Max Load) (MPa) | Bending Modulus (GPa) | Strain at Yield (MaxLoad) (mm/mm) |
|---|---|---|---|---|---|---|
| - | 100 | - | 49.4$\pm$2.9 | 85.1$\pm$4.2 | 2.2$\pm$0.2 | 0.062$\pm$0.002 |
| 37 | 53 | 10 | 184.2$\pm$4.2 | 318.4$\pm$10.6 | 14.9$\pm$0.4 | 0.023$\pm$0.001 |
| 38 | 52 | 10 | 220.5$\pm$31.0 | 366.5$\pm$56.7 | 17.4$\pm$1.9 | 0.023$\pm$0.001 |
| 48 | 44 | 8 | 287.7$\pm$18.1 | 420.0$\pm$39.1 | 21.5$\pm$0.4 | 0.022$\pm$0.003 |

**Example 2.**

**Matrix:** Poly-L/DL-lactide 70/30 ($PLA_{70}$), the same raw material from Boehringer Ingelheim, Germany, as in Example 1.

**Polymer fiber-reinforcement:** This was made of the same Poly-L/D-lactide 96/4 (from Purac Biochem, the Netherlands) as in Example 1.

**Glass fiber reinforcement:** Bioactive Glass 1-989898 fibers (diameter about 20-35$\mu$m) were manufactured at Tampere University of Technology (Institute of Biomaterials) as in Example 1.

**Polymer reinforcement used to bind BaG-fibers: PLGA 50/50,** the same raw material from Boehringer Ingelheim as in Example 1.

Test specimens having dimensions of about 50 x 10 x 1.5 mm were manufactured in same fashion as in Example 1. from preprocessed $PLA_{70}$ flat strips (48 wt-%), bioactive glass 1-98 (BaG) fibers (42 wt-%) and $PLA_{96}$ fibers (10 wt-%). The only significant difference here was that the PLA96 fibers were discontinuous. Circular shaped braids were cut from one side so that their final shape was a flat braid or sheet composed of discontinuous 10-15 mm long fibers.

The strength of the polymer composites reinforced with discontinuous fibers can reach the strength of continuous fiber composites when the fiber length is approximately 10* $I_c$, (where $I_c$ = critical fiber length) and is 90 % of the strength of 4*$I_c$ (D. Hull). The critical length $I_c$ of the fibers, which is defined as the minimum length of fiber required for stress to build up to the fracture strength ($\sigma_f^*$) of the fiber, is given by

$$I_c = r\sigma_f^*/\tau$$

where r = radius of the fiber, and $_\tau$ = the shear stress parallel to the fiber resisting pull-out (D. Hull).

The order of preforms which were laid into the mold (of Example 1):

**(BaG/$PLA_{96}$/$PLA_{70}$/BaG/$PLA_{70}$/$PLA_{96}$/BaG)**

The compression molding cycle for manufacturing the laminated specimens was identical to that of Example 1.The typical mechanical properties of samples for Example 2. (6 parallel samples) are shown in Table 2. The test specimens expressed fractures shortly after the maximum load but did not fragment. This behaviour was analogous with that of the samples in Example 1, while the reinforcing PLA96 fibers kept the fractured parts in position and prevented fragmentation.

**Table 2.**

| BaG fiber content wt-% | $PLA_{70}$ matrix content wt-% | $PLA_{96}$ fiber content wt-% | Load at Yield (Max load) (N) | Stress at Yield (Max Load) (MPa) | Modulus (GPa) | Strain at Yield (MaxLoad) (mm/mm) |
|---|---|---|---|---|---|---|
| 42 | 48 | 10 | 216.6±21.8 | 378.2±41.5 | 16.2±1.4 | 0.025±0.003 |

*Reference:* Hull D., An Introduction to Composite Materials, Cambridge University Press, Cambridge, UK, 1981, pp. 199-219.

**Example 3.**

**Matrix:** Poly-L/DL-lactide 70/30 ($PLA_{70}$), the same raw material from Boehringer Ingelheim, Germany, as above.

**Polymer fiber-reinforcement:** Poly-L/D-lactide 96/4, raw material from Purac Biochem, the Netherlands. Fibers were made as above.

**Glass fiber reinforcement:** Bioactive Glass 1-98 fibers (diameter about 20-35 $\mu$m) were manufactured at Tampere University of Technology (Institute of Biomaterials) as above.

**Polymer reinforcement used to bind BaG-fibers: PLGA 50/50,** the same raw material from Boehringer Ingelheim as in Example 1.

Test specimens having dimensions of about 50 x 10 x 2.6 mm were manufactured in the same fashion as in Example 1 from preprocessed $PLA_{70}$ flat strips (52 wt-%), bioactive glass 1-98 (BaG) fibers (43 wt-%) and $PLA_{96}$ fibers (5 wt-%). The BaG prepreg material was here about 2-3 times thicker (thickness about 0.65 mm) than in Example 1. and this thicker prepreg material was used only on the top and bottom surfaces of the test specimens, while the BaG layer in the middle of the laminate composite was the same prepreg material as used in Example 1. The polymer fiber reinforcement here was continuous and it was introduced into the composite structure by covering $PLA_{70}$ flat strips by $PLA_{96}$ braids as in Example 1.

The order of preforms on a compression mold was:

**(thick-BaG/($PLA_{96}$/$PLA_{70}$/$PLA_{96}$)/thin-BaG/($PLA_{96}$/$PLA_{70}$/$PLA_{96}$)/thick-BaG)**

The compression molding cycle was the same as in Example 1 and in Example 2.

Table 3. shows the typical mechanical properties of samples of this Experiment (2 parallel samples).

The test specimens expressed fractures shortly after the maximum load, but in the same way as in Example 1. and Example 2., the reinforcing $PLA_{96}$ fibers kept the fractured parts in position and prevented fragmentation. An interesting finding was that although the BaG-fiber content was here smaller than that in the strongest test specimens of Example 1., the bending modulus was much higher. This indicates that the structure of the composite strongly affects its mechanical properties.

**Table 3.**

| BaG fiber content wt-% | PLA$_{70}$ matrix content wt-% | PLA$_{96}$ fiber content wt-% | Load at Yield (Max load) (N) | Stress at Yield (Max Load) (MPa) | Modulus (GPa) | Strain at Yield (MaxLoad) (mm/mm) |
|---|---|---|---|---|---|---|
| 43 | 52 | 5 | 339.8±25.8 | 306.0±18.4 | 27.2±1.0 | 0.013±0.001 |

Example 4.

**Matrix:** Poly-L/DL-lactide 70/30 (PLA$_{70}$), raw material from Boehringer Ingelheim (Germany), RESOMER® LR 708, Lot No. 290358, initial Mw about 370 000 Da (I.V. 5.9 - 6.2), (when processed into the form of flat strips MW about 215 000 Da). Matrix was melt processed into the form of thin (about 0.5mm flat strips).

**Polymer fiber-reinforcement:** Poly-L/D-lactide 96/4, raw material from Purac Biochem (the Netherlands), PURASORB® PLD, Lot No. 0209000939, intial I.V. 5.48 dl/g (when processed into the form of fibers Mw about 150 000 Da, The fibers with final diameter of about 0.085-0.095mm were made by melt spinning with a single screw extruder. PLA96 fibres were in the form of circular braids composed of 16 separate fibre bundles (8-filaments on each.)

**Glass fiber reinforcement:** Bioactive Glass 13-93 (53.0% SiO$_2$, 6.0% Na$_2$O, 20.0% CaO, 4.0% P$_2$O$_5$ 12.0% K$_2$O, 5.0% MgO) fibers with the diameter of ca. 20-35 $\mu$m were manufactured at Tampere University of Technology (Institute of Biomaterials) by glass melt spinning. Bioactive Glass fibres were in the form of sheets, in which the fibres were bound together using a solution of PLA70 (RESOMER® LR 708, Lot No. 290358) and acetone.

Test specimens, sized about 50 x 10 x 1.5 mm were manufactured by means of compression molding from preprocessed PLA$_{70}$ flat strips (about 40 wt-%), bioactive glass 13-93 (BaG) fibers (about 40 wt-%) and PLA$_{96}$ fibers (about 20 wt-%). The manufacturing methods of the raw materials and the compression molding cycle of composites were similar as in the previous examples (1-3).

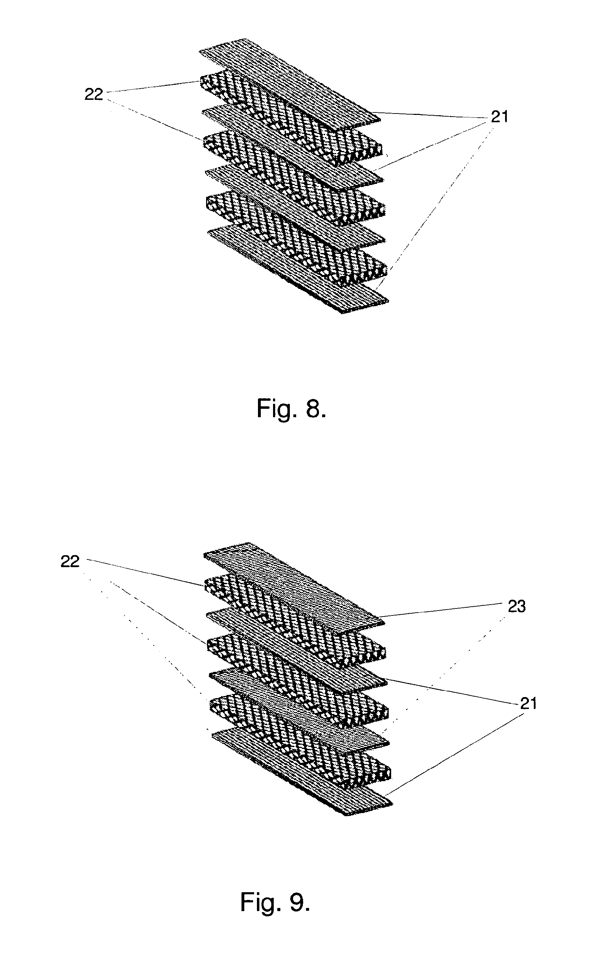

Three different compositions of BaG and PLA96 fibre reinforced composites were manufactured (Structures 1, 2 and 3, see figures 8-10), and a plate composed of pure PLA70 (Structure 4, not shown) was used as a reference material.

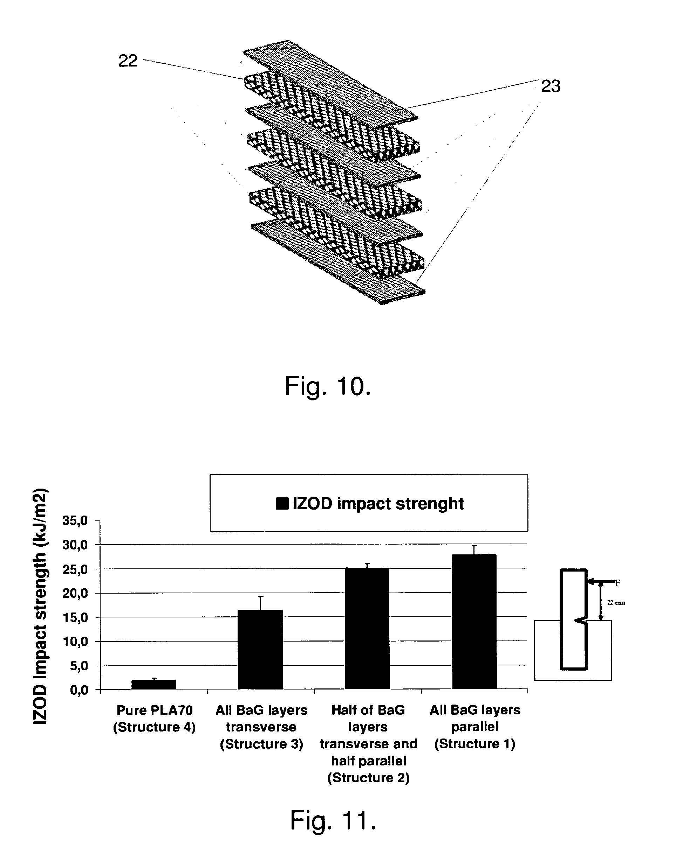

Structure 1, which is shown in Fig. 8, comprises bioactive glass fibre sheets 21 aligned parallel to the longitudinal axis and strips 22. The strips 22 comprise a flat strip of PLA70 and PLA96 fibers which cover the flat strip. The PLA96 may form for example a braiding which is pulled over the flat strip. The PLA96 fibers form an angle with the longitudinal direction of the strip 22. The angle may be approximately 45°. Structure 2, shown in Fig. 9, also comprises strips 22 and bioactive glass fiber sheets 21 but half of the bioactive glass fiber sheets (sheets 23) are traverse to the longitudinal axis. Structure 3, shown in Fig. 10, comprises strips 21 and bioactive glass fiber sheets 23 aligned traverse to the longitudinal axis.

The aim of this example was to analyze the effect of the direction of BaG fibre reinforcement in impact resistance of composite structures. The test method used was IZOD impact strength measurement outlined in standard ISO 180. The alignment of PLA96 fibre reinforcements was similar in every composite structure analyzed (PLA70 matrix flat strips were covered by PLA96 fibre reinforcement). The number of parallel test specimens was 4 for all structures analyzed.

The measurement of impact strength was made for notched test specimens and determined using the IZOD method according to international standard ISO 180 (ISO 180:2000. Plastics -Determination of Izod impact strength. International Organization of Standardization 2000. p 1-10).

The thickness was, however, smaller than that mentioned in the standard (4 mm in ISO 180 and about 1.5 mm on this example). The measurements were made using Ceast Resil 5,5 testing machine (Pianezza-Torino, Italy). The pendulum struck the notched side of the specimens. The striking edge of the pendulum was 22 mm above the top plane of the support. The dimensions of the test specimens were 1.5x10x50 mm, the notch was 2 mm deep and the apex angle was 45°. The impact strength ($J_{impact}$) was expressed in kilojoules per square metre (kJ m$^{-2}$) and calculated according to equation (1).

$$J_{impact} = \frac{E_{measured} - E_{hammer}}{bh} \qquad (1)$$

Where $E_{measured}$ is the measured energy of impact, $E_{hammer}$ is the energy of the hammer without specimen, b is the sample thickness and h is the sample width. The used testing machine calculated the value for $E_{measured}$ - $E_{hammer}$ automatically.

The results of impact strength measurements are presented in Figure 11 and in Table 4. In Fig. 11 one can also see (on the right hand side) the principle of the test.

Table 4. Results of IZOD impact strength measurements and percentual comparison to pure PLA70 and the effect of BaG fibre alignment.

| Structure | Impact Strength (kJ/m2) | Percentual increase in Impact resistance, compared to pure PLA70 (%) | Percentual increase in Impact resistance, compared to Structure 3 (%) | Percentual increase in Impact resistance, compared to Structure 2 (%) |
|---|---|---|---|---|
| Structure 4 (pure PLA70) | 1,8±0,4 | | | |
| Structure 3 (All BaG layers transverse) | 16,2±3,0 | 786 | | |
| Structure 2 (Half of BaG transverse and half parallel) | 25,0±1,1 | 1267 | 54 | |
| Structure 1 (All BaG layers parallel) | 27,7±2,1 | 1418 | 71 | 11 |

It can be seen in Figure 11 and in Table 4 that double fibre reinforcing (BaG and PLA96 fibres) increased the impact strength of composites substantially. The reinforcing effect was 786 to1418% depending on BaG fibre alignment when compared to test specimens composed of pure PLA70. The effect of BaG fibre alignment can also be seen clearly, as the impact strength was dependent on the alignment of BaG fibre-sheets; the impact resistance of Structure 1 was 54% higher than that of Structure 2 and 71 % higher than that of Structure 3. In other words, the highest values were measured for composites having a BaG fibre alignment parallel to the longitudinal axis of composites, and the lowest values for double fibre reinforced composites were measured for structures in which all sheets of BaG fibres were aligned transverse to the longitudinal axis. The difference between Structure 1 and Structure 2 was 11 %.

Besides the impact strength the fiber orientation has also another consequence. If all reinforcing fibers are parallel to each other and a fracture occurs in an implant, it will easily break apart. If there are fibers extending in different directions, the fracture cannot advance and the implant holds together. This is important because it has been reported that fragile ceramic implants have been broken in the system and have incurred the tetraplegia.

**Claims**

1. A bioabsorbable and bioactive composite material for surgical musculoskeletal applications comprising a bioabsorbable polymeric matrix material which is reinforced with bioabsorbable polymeric fibers and bioabsorbable ceramic fibers, **characterized in that**

   - the surgical bioabsorbable polymeric matrix material comprise layers which are laminated together and reinforced with the bioabsorbable polymeric fibers and the bioabsorbable ceramic fibers, the bioabsorbable polymeric fibers and the bioabsorbable ceramic fibers being longer than 150 $\mu$m and continuous in the layer, and
   - the superimposed layers comprise layers which differ from each other in their fiber orientation.

2. The composite material according to claim 1, **characterized in that** the amount of the bioabsorbable polymeric fibers is between 5 wt-% and 90 wt-% from the total weight of the composite.

3. The composite material according to claim 1 or 2, **characterized in that** the amount of the bioabsorbable ceramic fibers is between 10 wt-% and 90 wt-% from the total weight of the composite.

4. The composite material according to any preceding claim, **characterized in that** the polymeric matrix material is a homopolymer, a copolymer, a terpolymer, a polymer blend or a polymer alloy.

5. The composite material according to any preceding claim, **characterized in that** the bioabsorbable polymeric fibers are made of a homopolymer, or of a copolymer, or of a terpolymer, or of a polymer blend or alloy.

6. The composite material according to any preceding claim, **characterized in that** the bioabsorbable ceramic reinforcing fibers are made of calcium phosphate and/or of a bioactive glass.

7. The composite material according to any preceding claim, **characterized in that** the diameter of the bioabsorbable polymeric reinforcing fibers is between 4 $\mu$m and 800 $\mu$m, preferably between 20 $\mu$m and 500 $\mu$m.

8. The composite material according to any preceding claim, **characterized in that** the diameter of bioceramic fibers is between 2 $\mu$m and 500 $\mu$m, preferably between 20 $\mu$m and 200 $\mu$m.

9. The composite material according to any preceding claim, **characterized in that** the composite material contains at least one pharmaceutically active agent in the bioabsorbable polymer matrix and/or in the bioabsorbable polymeric fibers.


**Patentansprüche**

1. Bioabsorbierbares und bioaktives Verbundmaterial für chirurgische Muskel-Skelett-Anwendungen, umfassend ein bioabsorbierbares Polymermatrixmaterial, das mit bioabsorbierbaren Polymerfasern und bioabsorbierbaren Keramikfasern verstärkt ist, **dadurch gekennzeichnet, dass**

   - das chirurgische bioabsorbierbare Polymermatrixmaterial Schichten umfasst, die zusammenlaminiert und mit den bioabsorbierbaren Polymerfasern und den bioabsorbierbaren Keramikfasern verstärkt sind, wobei die bioabsorbierbaren Polymerfasern und die bioabsorbierbaren Keramikfasern länger als 150 $\mu$m und in der Schicht durchgehend sind, und
   - die übereinanderliegenden Schichten Schichten umfassen, die sich in ihrer Faserausrichtung voneinander unterscheiden.

2. Verbundmaterial nach Anspruch 1, **dadurch gekennzeichnet, dass** die Menge der bioabsorbierbaren Polymerfasern zwischen 5 Gew.-% und 90 Gew.-% des Gesamtgewichts des Verbundes beträgt.

3. Verbundmaterial nach Anspruch 1 oder 2, **dadurch gekennzeichnet, dass** die Menge der bioabsorbierbaren Keramikfasern zwischen 10 Gew.-% und 90 Gew.-% des Gesamtgewichts des Verbundes beträgt.

4. Verbundmaterial nach irgendeinem vorangehenden Anspruch, **dadurch gekennzeichnet, dass** das Polymermatrixmaterial ein Homopolymer, ein Copolymer, ein Terpolymer, eine Polymermischung oder eine Polymerlegierung ist.

5. Verbundmaterial nach irgendeinem vorangehenden Anspruch, **dadurch gekennzeichnet, dass** die bioabsorbierbaren Polymerfasern aus einem Homopolymer oder einem Copolymer oder einem Terpolymer oder einer Polymermischung oder -legierung bestehen.

6. Verbundmaterial nach irgendeinem vorangehenden Anspruch, **dadurch gekennzeichnet, dass** die bioabsorbierbaren verstärkenden Keramikfasern aus Calciumphosphat und/oder einem bioaktiven Glas bestehen.

7. Verbundmaterial nach irgendeinem vorangehenden Anspruch, **dadurch gekennzeichnet, dass** der Durchmesser der bioabsorbierbaren verstärkenden Polymerfasern zwischen 4 $\mu$m und 800 $\mu$m, vorzugsweise zwischen 20 $\mu$m und 500 $\mu$m liegt.

8. Verbundmaterial nach irgendeinem vorangehenden Anspruch, **dadurch gekennzeichnet, dass** der Durchmesser der Biokeramikfasern zwischen 2 $\mu$m und 500 $\mu$m, vorzugsweise zwischen 20 $\mu$m und 200 $\mu$m liegt.

9. Verbundmaterial nach irgendeinem vorangehenden Anspruch, **dadurch gekennzeichnet, dass** das Verbundmaterial mindestens ein pharmazeutisch aktives Mittel in der bioabsorbierbaren Polymermatrix und/oder in den bioabsorbierbaren Polymerfasern enthält.

**Revendications**

1. Matériau composite bioabsorbable et bioactif pour des applications musculosquelettiques chirurgicales comprenant un matériau matriciel polymérique bioabsorbable qui est renforcé avec des fibres polymériques bioabsorbables et des fibres céramiques bioabsorbables, **caractérisé en ce que**

   - le matériau matriciel polymérique bioabsorbable chirurgical comprend des couches qui sont stratifiées ensemble et renforcées avec les fibres polymériques bioabsorbables et les fibres céramiques bioabsorbables, les fibres polymériques bioabsorbables et les fibres céramiques bioabsorbables étant plus longues que 150 $\mu$m et continues dans la couche, et
   - les couches superposées comprennent des couches qui diffèrent les unes des autres dans leur orientation de fibres.

2. Matériau composite selon la revendication 1, **caractérisé en ce que** la quantité des fibres polymériques bioabsorbables est entre 5% en poids et 90% en poids du poids total du composite.

3. Matériau composite selon la revendication 1 ou 2, **caractérisé en ce que** la quantité des fibres céramiques bioabsorbables est entre 10% en poids et 90% en poids du poids total du composite.

4. Matériau composite selon une quelconque revendication précédente, **caractérisé en ce que** le matériau matriciel polymérique est un homopolymère, un copolymère, un terpolymère, un mélange de polymères ou un alliage de polymères.

5. Matériau composite selon une quelconque revendication précédente, **caractérisé en ce que** les fibres polymériques bioabsorbables sont constituées d'un homopolymère, ou d'un copolymère, ou d'un terpolymère, ou d'un mélange ou d'un alliage de polymères.

6. Matériau composite selon une quelconque revendication précédente, **caractérisé en ce que** les fibres de renfort céramiques bioabsorbables sont constituées de phosphate de calcium et/ou d'un verre bioactif.

7. Matériau composite selon une quelconque revendication précédente, **caractérisé en ce que** le diamètre des fibres de renfort polymériques bioabsorbables est entre 4 $\mu$m et 800 $\mu$m, de préférence entre 20 $\mu$m et 500 $\mu$m.

8. Matériau composite selon une quelconque revendication précédente, **caractérisé en ce que** le diamètre des fibres biocéramiques est entre 2 $\mu$m et 500 $\mu$m, de préférence entre 20 $\mu$m et 200 $\mu$m.

9. Matériau composite selon une quelconque revendication précédente, **caractérisé en ce que** le matériau composite contient au moins un agent pharmaceutiqement actif dans la matrice polymérique bioabsorbable et/ou dans les fibres polymériques bioabsorbables.

Fig. 1a.

Fig. 1b.

Fig. 2a.

Fig. 2b.

Fig. 2c.

Fig. 3.

Fig. 4.

Fig. 5.

Fig. 6a.

Fig. 6b.

Fig. 7.

Fig. 8.

Fig. 9.

22

23

Fig. 10.

IZOD impact strenght

IZOD Impact strength (kJ/m2)

35,0
30,0
25,0
20,0
15,0
10,0
5,0
0,0

Pure PLA70
(Structure 4)

All BaG layers
transverse
(Structure 3)

Half of BaG
layers
transverse and
half parallel
(Structure 2)

All BaG layers
parallel
(Structure 1)

F

22 mm

Fig. 11.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4898186 A
- US 1990 A
- EP 1009448 B1
- WO 2004049904 A

### Non-patent literature cited in the description

- **S. VAINIONPÄÄ ; P. ROKKANEN ; P. TÖRMÄLÄ.** Surgical Applications of Biodegradable Polymers in Human Tissue. *Progress in Polymer Science,* 1989, vol. 14, 679-716
- **J. EITENMÜLLER ; K.L. GERLACH ; T. SCHMICKAL ; H. KRAUSE.** An in Vivo Evaluation of a New High Molecular Weight Polylactide Osteosynthesis Device. *European Congress on Biomaterials,* 14 September 1986, 94
- **P. TÖRMÄLÄ.** Biodegradable Self-Reinforced Composite Materials; Manufacturing, Structure and Mechanical Properties. *Clinical Materials,* 1992, vol. 10, 29-34
- **S.M. SNYDER ; E. SCHNEIDER.** Estimation of Mechanical Properties of Cortical Bone by Computed Tomography. *Journal of Orthopedic Research,* 1991, vol. 9, 422-431
- **O.H. ANDERSSON ; K.H. KARLSSON.** Bioactive Glass, Biomaterials Today and Tomorrow. *Proceedings of the Finnish Dental Society Days of Research,* 10 November 1995, 15-16
- Advanced Bone Cement for Long Term Orthopaedic Applications. **J.C. BEHIRI ; M. BRADEN ; S.N. KHORASHANI ; D. WIWATTANADATE ; W. BONFIELD.** Bioceramics. Butterworth-Heinemann Ltd, 1991, vol. 4, 301-307
- **M. ZIMMERMAN ; T. GUASTAVIN ; J.R. PARSONS ; H. ALEXANDER ; T.C. LIN.** The in vivo biocompatibility and in vitro degradation of absorbable glass fiber reinforced composites. *12th Ann. Meeting Soc. Biomater.,* 1986, 16
- **A. SAIKKU-BÄCKSTRÖM et al.** *J. Mater. Sci: Mater. Med.,* 1999, vol. 10, 1-8
- In Vitro and in Vivo Evaluation of Polyhydroxybutyrate and of Polyhydroxyvalerate Reinforced with Hydroxyapatite. *Biomaterials,* 1991, vol. 12, 841-847
- **E. SUOKAS.** Processing, microstructure and properties of thermotropic liquid crystalline polymers and their carbon fibre composites. Tampere University of Technology, Publication 267, 1999, 269
- **M. KELLOMÄKI et al.** *13th Eur. Conf. Biom.,* 04 September 1997, 90
- **HULL D.** An Introduction to Composite Materials. Cambridge University Press, 1981, 199-219